# EUROPÄISCHE PATENTSCHRIFT

(11) **EP 0 789 512 B1**
(45) Veröffentlichungstag und Bekanntmachung des Hinweises auf die Patenterteilung: **07.01.1999**
(21) Anmeldenummer: 95937023.0
(22) Anmeldetag: 28.10.1995
(51) Int. Cl.: A01N 35/02, A01N 31/02

(54) **ZUR BEKÄMPFUNG VON HYLOTRUPES BAJULUS UND PYRRHIDIUM SANGUINEUM GEEIGNETE MISCHUNGEN UND MITTEL**
MIXTURES AND AGENTS SUITABLE FOR COMBATTING HYLOTRUPES BAJULUS AND PYRRHIDIUM SANGUINEUM
MELANGES ET AGENTS APPROPRIES A LA LUTTE CONTRE HYLOTRUPES BAJULUS ET PYRRHIDIUM SANGUINEUM

(30) Priorität: 03.11.1994 DE 4439223
(43) Veröffentlichungstag der Anmeldung: 20.08.1997
(73) Patentinhaber: BASF AKTIENGESELLSCHAFT, 67056 Ludwigshafen (DE)
(72) Erfinder: FETTKOETTER, Regine, D-95447 Bayreuth (DE); DETTNER, Konrad, D-95448 Bayreuth (DE); NOLDT, Uwe, D-21502 Geesthacht (DE); SCHRÖDER, Frank, D-22309 Hamburg (DE); WITTKO, Franke, D-21465 Reinbek (DE)
(86) Internationale Anmeldenummer: EP9504236
(87) Internationale Veröffentlichungsnummer: WO9613976

(56) Entgegenhaltungen:
- DE-A- 2 254 697
- EXPERIENTIA, Bd. 51, Nr. 3, 15.März 1995 BASEL CH, Seiten 270--277, R. FETTKÖTHER ET AL. 'The male pheromone of the old house borer Hylotrupes bajulus: identification and female response.' in der Anmeldung erwähnt
- JUSTUS LIEBIGS ANNALEN DER CHEMIE, Nr. 12, Dezember 1994 WEINHEIM DE, Seiten 1211-1218, F. SCHRÖDER ET AL. 'Synthesis of (3R)-3-hydroxy-2-hexanone, (2R,3R)-2,3-hexanediol and (2S,3R)-2,3-hexanediol, the male sex pheromone of Hylotrupes bajulus and Pyrrhidium sanguineum.' in der Anmeldung erwähnt
- CENTRAL PATENTS INDEX, BASIC ABSTRACTS JOURNAL Week 8245 Derwent Publications Ltd., London, GB; AN 96013E & JP,A,57 158 709 (SUN CHEMICAL CORP.) , 30.September 1982

## Beschreibung

Die vorliegende Erfindung betrifft zur Bekämpfung von Hylotrupes bajulus und Pyrrhidium sanguineum geeignete Mischungen und Mittel sowie deren Verwendung hierzu.

In Fällen, in denen die Bekämpfung von Schadinsekten mit herkömmlichen Insektiziden nicht angezeigt ist, etwa bei der Behandlung von Bauholz in Wohngebäuden, besteht ein großes Interesse an einer biologischen Schädlingsbekämpfung, welche sich selektiv gegen eine einzige Schädlingsart oder eine kleine Gruppe von Schädlingen richtet.

Verfahren zur Schädlingsbekämpfung, bei denen man sich der Sexuallockstoffe oder Pheromone (im folgenden "Lockstoffe") der jeweiligen Schädlinge bedient, sind allgemein bekannt (vgl. die DE-A 36 03 377 [Bekämpfung des bekreuzten Traubenwicklers] oder die WO-A 93/08148 [Bekämpfung des Apfelwicklers]).

Vor allem Hylotrupes bajulus und daneben Pyrrhidium sanguineum sind bedeutende Holzschädlinge, weil sich ihre Larven in Holz einbohren und es zerstören.

Hylotrupes bajulus und Pyrrhidium sanguineum gehören zur Familie der Cerambycidae (Bockkäfer), bei denen die Beteiligung von Lockstoffe im Paarungsverhalten bereits seit geraumer Zeit untersucht wird. Kontaktpheromone bzw. Pheromone, die auf kurze Distanzen wirken (short-range pheromones), wurden bereits bei Weibchen verschiedener Bockkäferarten gefunden (vgl. USDA Forest Service Research Note NE-240, 1 (1977); Appl. Ent. Zool. 27, Seite 489 (1992); J. Chem. Ecol. 18, Seite 245 (1992); ibid., 19, Seite 2347 (1993)). Bei Hylotrupes bajulus wurde vermutet, daß die Weibchen auf ihren Elytren einen Lockstoff bilden, welcher der Paarfindung dient.

Über einen Lockstoff, der von den Männchen abgegeben wird, liegt eine Studie vor (vgl. Chem. Lett., Seite 263 (1984); Appl. Ent. Zool. 17, Seite 497 (1982); ibid. 21, Seite 606 (1986)).

In der Regel macht es unter wirtschaftlichen Gesichtspunkten wenig Sinn, zur Schädlingsbekämpfung die natürlichen Lockstoffe aus den sie produzierenden Schädlingen zu isolieren, weil sie dort nur in geringer Konzentration vorliegen. Stattdessen richtet sich die Suche meist auf naturidentische synthetische, oder mit diesen strukturell verwandte synthetische Stoffe.

Der vorliegenden Erfindung lag daher die Bekämpfung von Hylotrupes bajulus und Pyrrhidium sanguineum mittels synthetischer Lockstoffe als Aufgabe zugrunde, welche entweder naturidentisch, oder chemisch-strukturell mit den natürlichen Lockstoffen verwandt sind.

Demgemäß wurde eine Mischung gefunden, welche ein Stereoisomeres von 3-Hydroxyhexan-2-on und zwei Stereoisomere von Hexan-2,3-diol enthält.

Ferner wurden Mischungen gefunden, in denen die vorstehend genannten Stereoisomeren spezifische Konfigurationen der asynmetrischen Kohlenstoffatome aufweisen sowie weiterhin in bestimmten Mengenverhältnissen nebeneinander vorliegen.

Des weiteren betrifft die Erfindung Mittel auf Basis der vorstehend genannten Mischungen und außerdem die Verwendung der Mischungen, Mittel sowie von (3R)-3-Hydroxyhexan-2-on zur Bekämpfung von Hylotrupes bajulus und Pyrrhidium sanguineum.

Grundsätzlich gibt es drei verschiedene Möglichkeiten, Lockstoffe im Pflanzen- und Materialschutz anzuwenden:

### 1. Monitortechnik

Sogenannte Pheromonfallen, bestückt mit synthetischen Lockstoffködern, werden in potentiellen Befallsgebieten ausgehängt. Der Fallenfang von weiblichen Käfern erbringt den Nachweis des Auftretens des Schädlings. Außerdem lassen sich Hinweise zur Befallsstärke und auf den richtigen Zeitpunkt der Bekämpfung ableiten.

### 2. Abfangtechnik

Man kann den Lockstoff mit insektiziden Wirkstoffen kombinieren. Es besteht die Möglichkeit, dem Köder bzw. der Falle Insektizide zuzusetzen oder aber nur in unmittelbarer Umgebung der Falle zu behandeln, damit dann der größte Teil der aus weiter Entfernung angelockten weiblichen Käferbevölkerung abgetötet werden kann. Die Biotopbelastung ist auf ein vertretbares Maß reduziert.

### 3. Paarungsstörungsmethode

Schließlich kann der Schädling durch das Verfahren der Luftraumsättigung mit Lockstoffen oder ähnlich wirkenden Substanzen bekämpft werden. Die weiblichen Käfer werden am Auffinden der Männchen gestört. Dadurch wird die Paarung der Tiere verhindert. In diesem Fall wird im gesamten Bereich der zu schützenden Pflanzenkultur eine größere Menge des Lockstoffes im Luftraum verteilt, so daß die Weibchen überall die Gegenwart des Duftstoffes empfinden können und damit ihr normales Orientierungsverhalten gestört ist.

Vor allem bei der dritten Methode (Paarungsstörungsmethode) handelt es sich um eine äußerst selektive und auch effektive Möglichkeit zur Bekämpfung einer unerwünschten Spezies unter Schonung der Nicht-Zielorganismen, insbesondere aller Nützlinge.

Auch nach dieser Methode werden nur verhältnismäßig kleine Mengen der Wirkstoffe, welche oft nur Bruchteile der üblichen Dosen der klassischen Insektizidwirkstoffe entsprechen, benötigt (vgl. Birch (ed.), Pheromones, North Holland Publ. Co., 1974).

Die Methoden 1 und 2 sind insofern nachteilig, als der Lockstoff synthetischer Herkunft seinem natürlichen Vorbild bezüglich Struktur und Reinheit exakt gleichen muß (Minks und Voermann, Entomologia exp. and appl. 16, Seiten 341 bis 349 (1973) sowie Wegler, Chemie der Pflanzenschutz- und Schädlingsbekämpfungsmittel Bd. 6, S. 167 (1981)). Technische Gemische o.ä. haben bei Fallenfangversuchen regelmäßig versagt.

Bei der erfindungsgemäßen Bekämpfung von Hylotrupes bajulus und Pyrrhidium sanguineum kann man sich aller drei Techniken bedienen.

Es wurde gefunden, daß die Männchen von Hylotrupes bajulus und Pyrrhidium sanguineum Lockstoffe für ihre Weibchen produzieren, welche essentiell (3R)-3-Hydroxyhexan-2-on, (2S,3R)-Hexan-2,3-diol und (2R,3R)-Hexan-2,3-diol und gegebenenfalls Hexan-2,3-dion und/oder racemisches 2-Hydroxyhexan-3-on enthalten (vgl. R. Fettköther, K. Dettner, F. Schröder, H. Meyer, W. Francke und U. Noldt in Experientia, 1994, im Druck; F. Schröder, U. Noldt, R. Fettköther, K. Dettner, W.A. König und W. Francke in Liebigs Ann. Chem., 1994, im Druck).

Es wurde weiterhin gefunden, daß sich für die Methode der Paarungsstörung bei Hylotrupes bajulus bzw. Pyrrhidium sanguineum Mischungen eignen, welche ein beliebiges Stereoisomeres von 3-Hydroxyhexan-2-on und zwei beliebige Stereoisomere von Hexan-2,3-diol enthalten oder in denen diese Stereoisomeren nur teilweise in der natürlich vorkommenden stereospezifischen Form vorliegen.

Bevorzugt ist bei der Bekämpfung der genannten Bockkäfer mittels der Paarungsstörungsmethode, und essentiell bei der Abfangtechnik oder der Monitortechnik, daß man als 3-Hydroxyhexan-2-on (3R)-3-Hydroxyhexan-2-on und/oder als Stereoisomere von Hexan-2,3-diol (2S,3R)-Hexan-2,3-diol und (2R,3R)-Hexan-2,3-diol verwendet.

Gegen beide Schadkäfer hat sich eine Mischung als wirksam erwiesen, welche
a) 70 bis 100 Gewichtsteile (3R)-3-Hydroxyhexan-2-on,
b) 0,01 bis 7, vor allem 3 Gewichtsteile (2R,3R)-Hexan-2,3-diol,
c) 0,01 bis 20, vor allem 10 Gewichtsteile (2S,3R)-Hexan-2,3-diol
   und gewünschtenfalls
d) 0,01 bis 5 Gewichtsteile Hexan-2,3-dion und/oder
e) 0,01 bis 7 Gewichtsteile eines racemischen Gemisches von 2-Hydroxyhexan-3-on
   enthält.

Vorteilhafterweise weist dabei das (3R)-3-Hydroxyhexan-2-on gegenüber seinem Enantiomeren einen Überschuß ("ee-Wert") von 70 bis 100, insbesondere von 90 bis 100 % auf.

Weiterhin ist von Vorteil, wenn das (2S,3R)-Hexan-2,3-diol gegenüber seinem Enantiomeren einen Überschuß von 20 bis 50, vor allem 30 bis 40 % und das (2R,3R)-Hexan-2,3-diol einen Überschuß von 15 bis 45, insbesondere von 25 bis 35 % aufweist, wobei vor allem bei Hylotrupes bajulus ferner ein Verhältnis von (2S,3R)-Hexan-2,3-diol zum diastereomeren (2R,3R)-Hexan-2,3-diol von 3:1 bis 4:1 bevorzugt ist.

Es wurde auch gefunden, daß (3R)-3-Hydroxyhexan-2-on bei der Paarungsstörungs-, der Abfang- und der Monitortechnik für sich allein wirksam ist. Teil der Erfindung ist auch, daß sich schon allein Mischungen zweier beliebiger Stereoisomeren von Hexan-2,3-diol für die Paarungsstörungsmethode und Mischungen von (2S,3R)-Hexan-2,3-diol und (2R,3R)-Hexan-2,3-diol für die Abfang- bzw. die Monitormethode eignen.

Was dabei die Mischungen von (2S,3R)-Hexan-2,3-diol und (2R,3R)-Hexan-2,3-diol angeht, so sind Gewichtsverhältnisse von 10 zu 1 bis 1 zu 1 bevorzugt und von 5 zu 1 bis 2 zu 1 besonders bevorzugt. Ferner weist in ihnen das (2S,3R)-Isomere vorzugsweise einen ee-Wert von 20 bis 60, und das (2R,3R)-Isomere einen solchen von 20 bis 60 auf.

Bei Hylotrupes bajulus hat sich als Lockstoff eine Mischung als besonders wirksam erwiesen, welche
a) 70 bis 80 Gewichtsteile (3R)-3-Hydroxyhexan-2-on, vor allem mit einem ee-Wert von 95 bis 100 %,
b) 4 bis 7 Gewichtsteile (2R,3R)-Hexan-2,3-diol, vor allem mit einem ee-Wert von 30 bis 40 %,
c) 14 bis 19 Gewichtsteile (2S,3R)-Hexan-2,3-diol, vor allem mit einem ee-Wert von 25 bis 35 % und
d) 1 bis 4 Gewichtsteile Hexan-2,3-dion und
e) 0,5 bis 4 Gewichtsteile eines racemischen Gemisches von 2-Hydroxyhexan-3-on
   enthält.

Es hat sich dabei herausgestellt, daß die Anwesenheit der Komponenten (d) und (e) für die Wirkung dieser als Lockstoff geeigneten Mischung gegenüber den Weibchen von Hylotrupes bajulus nicht essentiell ist.

Weiterhin wurde bei Pyrrhidium sanguineum eine besonders wirksame Mischung folgender Zusammensetzung ermittelt:
a) 92 bis 100 Gewichtsteile (3R)-3-Hydroxyhexan-2-on, vor allem mit einem ee-Wert von 95 bis 100 %,
b) 0,01 bis 1 Gewichtsteile (2R,3R)-Hexan-2,3-diol, vor allem mit einem ee-Wert von 85 bis 95 %,
c) 0,01 bis 1 Gewichtsteile (2S,3R)-Hexan-2,3-diol, vor allem als Racemat und
d) 0,5 bis 7 Gewichtsteile eines racemischen Gemisches von 2-Hydroxyhexan-3-on.

Die Anwesenheit der Komponente (d) ist für die Wirkung dieser Mischung gegenüber den Weibchen von Pyrrhidium sanguineum nicht essentiell. Die vorstehend genannten besonders wirksamen Mischungen eignen sich im besonderen Maße für die Monitor- und die Abfangtechnik.

Die als Lockstoffe geeigneten Mischungen sind durch organischchemische Synthese erhältlich, indem man die Komponenten, d.h. die Einzelstoffe oder, insbesondere bei Racematen, Stoffgemische der jeweils erforderlichen (stereo)chemischen Reinheit herstellt und dann im entsprechenden Verhältnis miteinander mischt.

Vorzugsweise arbeitet man so, daß man (3R)-3-Hydroxyhexan-2-on, (2S,3R)-Hexan-2,3-diol und (2R,3R)-Hexan-2,3-diol mit ee-Werten bzw. de-Werten > 95 % stereoselektiv herstellt und mit racemischen Gemischen von 3-Hydroxyhexan-2-on bzw. Hexan-2,3-diol in einem Verhältnis mischt, dessen Berechnung der Fachmann routinemäßig vornimmt, und gewünschtenfalls die vorgegebene Menge an Hexan-2,3-dion bzw. racemischem 2-Hydroxyhexan-3-on zusetzt.

Die Synthese dieser Einzelstoffe und Racemate erfolgt gemäß den folgenden Schemata.

### (3R)-3-Hydroxyhexan-2-on:

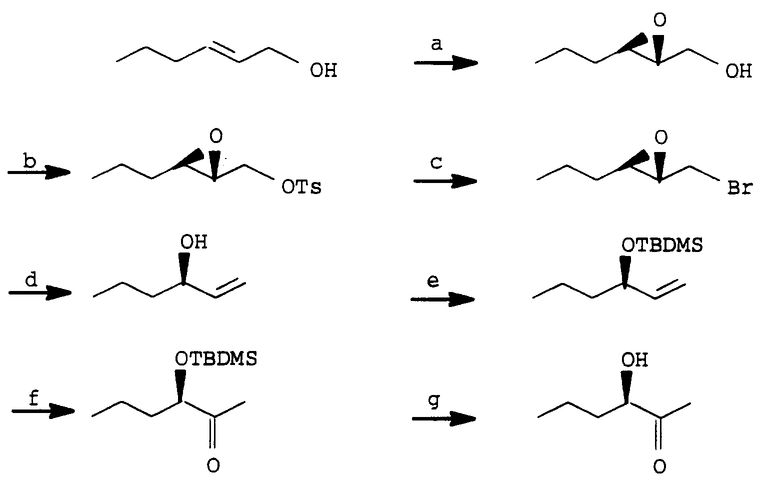

Ts = Toluol-4-sulfonylrest; TBDMS = tert.-Butyldimethylsilylrest
a: tert.-Butylhydroperoxid, Di-iso-propyl-D-(-)-tartrat, Titan-(IV)-iso-propylat
b: p-Toluolsulfonylchlorid, Kaliumhydroxid
c: Lithiumbromid
d: Zink, Natriumiodid
e: tert.-Butyldimethylsilylchlorid, Imidazol
f: Palladium-(II)-chlorid, Kupfer-(I)-chlorid, O₂
g: Fluorwasserstoffsäure

### (2S,3R)-Hexan-2,3-diol:

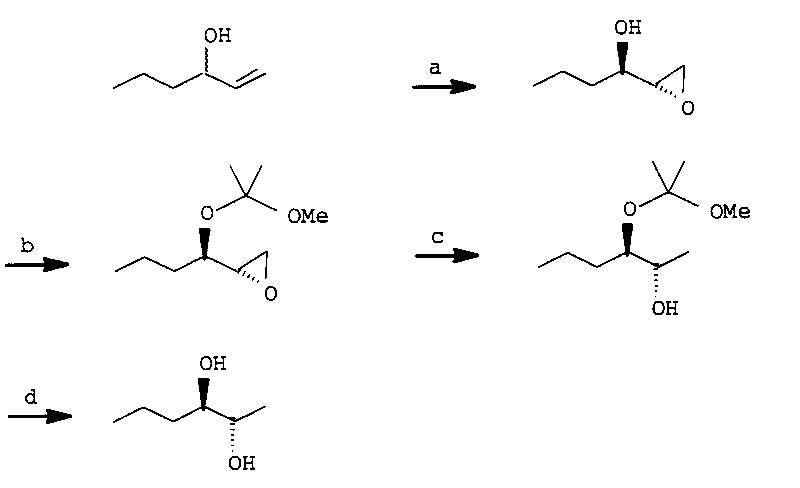

a: tert.-Butylhydroperoxid, Di-iso-propyl-D-(-)-tartrat, Titan-(IV)-iso-propylat
b: p-Toluolsulfonylchlorid, Kaliumcarbonat, 2-Methoxypropen
c: Lithiumaluminiumhydrid
d: Salzsäure

### (2R, 3R)-Hexan-2,3-diol:

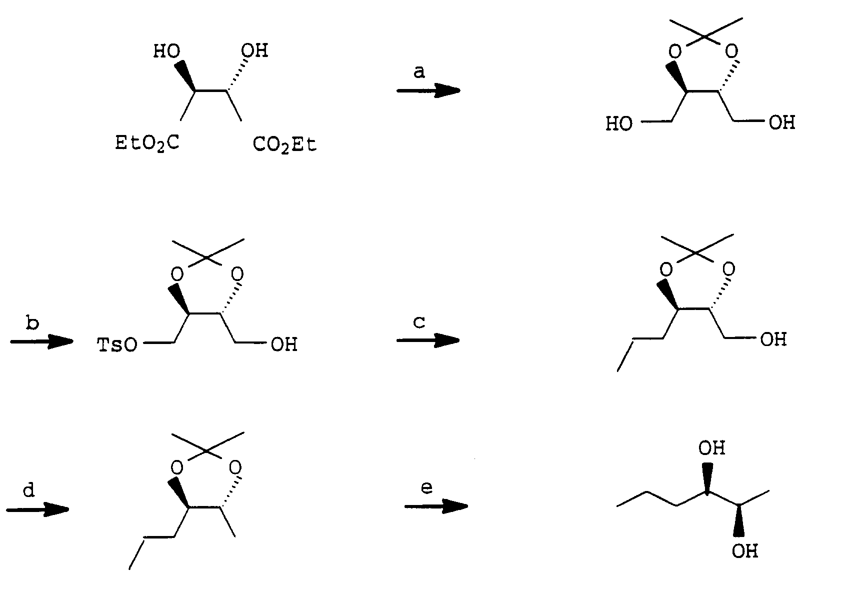

a: vgl. J. Org. Chem. 33, Seite 2171 (1968)
b: n-Butyllithium, p-Toluolsulfonylchlorid
c: Ethylmagnesiumbromid, Kupfer-(I)-bromid/Dimethylsulfid
d: p-Toluolsulfonylchlorid, Kaliumhydroxid; Lithiumaluminiumhydrid
e: Salzsäure

### Racemate von 3-Hydroxyhexan-2-on, 2-Hydroxyhexan-3-on und Hexan-2,3-diol:

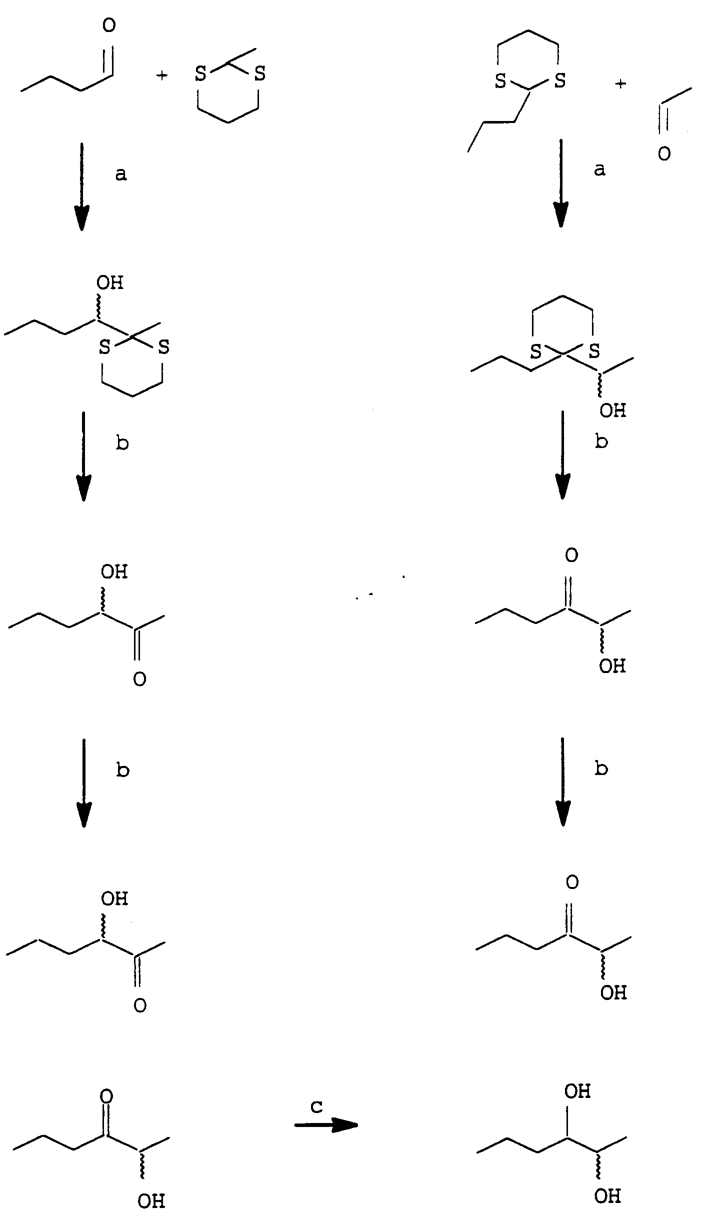

a: n-Butyllithium
b: N-Iodsuccinimid
c: Lithiumaluminiumhydrid

Hexan-2,3-dion ist kommerziell erhältlich.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Zu Vergleichszwecken wurden aus den Männchen von Hylotrupes bajulus bzw. Pyrrhidium sanguineum auch natürliche Lockstoffe gewonnen. Dies erfolgte mittels der sogenannten "Closed Loop Stripping Analysis" (CLSA), bei der die Lockstoffe in einer geschlossenen Vorrichtung mittels eines Gasstroms von den Insekten weggetragen, auf Aktivkohle adsorbiert, und aus dieser mittels eines organischen Lösungsmittel extrahiert werden (vgl. Analysis of Volatiles (Hrsg.: Schreier, P.), Verlag de Gruyter, Berlin, 1984 und Journal of High Resolution Chromatography and Chromatography Communications 7, 492-494 (1984)).

Als Lösungsmittel eignen sich hierbei Kohlenwasserstoffe, Halogenkohlenwasserstoffe, Alkohole, Ether und Ester oder Gemische dieser Lösungsmittel. Vorzugsweise verwendet man Lösungsmittel mit Siedepunkten unterhalb derer der Lockstoffkomponenten und insbesondere n-Pentan, n-Hexan, Methanol oder Dichlormethan bzw. deren Gemische.

Die erfindungsgemäßen Mischungen können zusammen mit üblichen Hilfsstoffen, z.B. entsprechend präparierten Streifen aus Kunststoff, Bindegarnen, lockstoffgefüllten Ampullen o.ä. (beispielsweise gemäß der älteren deutschen Anmeldung P 41 01 878.8) angewendet werden und auch herstellungsbedingte Verunreinigungen enthalten.

Zur Formulierung der Mischung kommen sowohl flüssige wie auch feste Präparationen in Frage. Als Lösungsmittel kommen hochsiedende, aromatische, aliphatische oder cycloaliphatische Verbindungen in Betracht. Neben Kohlenwasserstoffen eignen sich Ester, Ether oder Ketone besonders gut. Typische Vertreter dieser Klassen sind z.B. Xylol, Methylnaphthaline, Paraffinöle, Cyclohexanon, Ethylenglykolacetat, Isophoron und Dibutylphthalat. Diese Lösungsmittel können allein oder in Mischungen mit anderen Komponenten Verwendung finden. Weiterhin können Lösungen in pflanzlichen, tierischen oder synthetischen Ölen oder Fetten und anderen verdunstungshemmenden Lösungsmitteln mit niedrigem Dampfdruck wie z.B. Dioctylphthalat zum Zwecke der Wirkungsverlängerung hergestellt werden.

Des weiteren ist es möglich, die Mischung in oder an natürliche oder synthetische feste Träger wie Gummi, Kork, Zellulose, Kunststoffe, gemahlene Kohle, Holzmehl, Silikate, Bimskies, gebrannten Ton oder ähnliche feste Trägerstoffe zu binden oder in speziellen Kapselformulierungen oder Kunststoffbehältern einzusetzen, um so eine gleichmäßige Abgabe an die Luft über längere Zeiträume hinweg zu erreichen. Außerdem kann der Lockstoff aus geeigneten Behältern, z.B. Kapillaren oder anderen Gefäßen, durch enge Öffnungen oder durch Diffusion durch die Behälterwand sowie aus mehrschichtigen Kunststoffplättchen, sogenannte Flakes, zur Verdunstung gebracht werden, wodurch über längere Zeiträume hinweg besonders gleichmäßige Duftkonzentrationen erzielt werden.

Der Gehalt dieser Zubereitungen an der Mischung kann innerhalb weiter Grenzen schwanken. In Kapselformulierungen oder anderen geeigneten Behältern kann das Verhältnis Wirkstoff Zusatzstoff z.B. im Bereich von 10:1 bis 1:10³ liegen. In Kapselformulierungen oder anderen geeigneten Behältern kann z.B. der Wirkstoff in reiner, unverdünnter Form angewendet werden und sein Gewichtsanteil, bezogen auf die Gesamtformulierung, sehr hoch sein und bis zu 90 % betragen. Im allgemeinen genügen jedoch sehr geringe Wirkstoffkonzentrationen in den Zubereitungen, um die gewünschte Wirkung auf die Weibchen von Hylotrupes bajulus und/oder Pyrrhidium sanguineum auszuüben. Bevorzugt ist ein Mengenverhältnis Wirkstoff:Zusatzstoff von 1:3 bis 1:10² insbesondere 1:10 bis 1:100.

Ein besonderer Vorzug der erfindungsgemäßen Mischungen und Mittel besteht darin, daß ihre Anwendung in der Regel den Gebrauch üblicher sythetischer Insektizide entbehrlich macht. Der Mitverwendung der letzteren steht jedoch nach den bisherigen Erkenntnissen aus technischer Sicht nichts entgegen.

### Beispiele

### (Säulenchromatographie: Flash-Chromatographie an Kieselgel (Kieselgel 60, 240-400 mesh der Fa. Merck, Darmstadt, Deutschland); Gaschromatographie: Fisons GC 8000, split injector, FID, Wasserstoff als Trägergas)

### Beispiel 1

### Herstellung von (3R)-3-Hydroxyhexan-2-on

### a) (2R,3R)-2,3-Epoxyhexan-1-ol

Gepulvertes aktiviertes Molekularsieb (4 Å, 6 g), 2,13 g (7,5 mmol) Titan-(IV)-iso-propylat und 27 g (300 mmol) tert.-Butylhydroperoxid wurden nacheinander einer Lösung von Di-iso-propyl-D-(-)-tartrat (2,11 g, 9 mmol) in trockenem Dichlormethan (300 ml) bei -20°C unter Argon zugesetzt. Die Mischung wurde noch 20 Minuten bei -20°C gerührt. Danach wurden innerhalb von 30 Minuten 15 g (150 mmol) frisch destilliertes (E)-2-Hexen-1-ol in 70 ml trockenem Dichlormethan zugefügt. Die Mischung wurde noch 3 Stunden bei -20°C gerührt. Anschließend wurde sie langsam in eine eiskalte Lösung von 45 g (0,3 mol) Weinsäure und 50 g (0,18 mol) Eisen-(II)-sulfat-Heptahydrat in 300 ml Wasser ausgegossen. Die so entstandene Mischung wurde 20 Minuten lang kräftig gerührt. Die organische Phase wurde abgetrennt, und die Wasserphase wurde zweimal mit jeweils 100 ml Dichlormethan extrahiert. Die vereinigten organischen Phasen wurden mit Wasser und konzentrierter Kochsalzlösung gewaschen, über wasserfreiem Natriumsulfat getrocknet, und man entfernte danach das Lösungsmittel. Es folgte eine Chromatographie über 900 g Kieselgel (30-70 Vol.-% Ethylacetat in n-Hexan). Die Ausbeute an (2R,3R)-2,3-Epoxyhexan-1-ol betrug nach Destillation 88,7 % (Kp.: 85°C/14 hPa; gelbes Öl; [α]_{D}²¹ = +45,6 (c = 2,0 in Trichlormethan); Literatur: [α]_{D}²⁵ = -46,3° (c = 3,87 in Trichlormethane) für das Enantiomere (vgl. J. Am. Chem. Soc. 109, Seite 5765 (1987)).

### b) (2R,3R)-2,3-Epoxyhexyl-p-toluolsulfonat

26 g gepulvertes Kaliumhydroxid (0,45 mol) und 14,3 g (75 mmol) p-Toluolsulfonylchlorid wurden nacheinander bei -10°C zu einer gerührten Lösung von (2R,3R)-2,3-Epoxy-1-hexanol (8 g, 69 mmol) in Diethylether gegeben. Nach 5 Stunden bei 0°C wurde das Reaktionsgemisch in 200 ml Wasser ausgegossen. Die organische Schicht wurde abgetrennt und die wäßrige Phase zweimal mit jeweils 100 ml Diethylether extrahiert. Die vereinigten organischen Phasen wurden mit konzentrierter Kochsalzlösung gewaschen und über wasserfreiem Natriumsulfat getrocknet. Nach Entfernen des Lösungsmittels verblieb (2R,3R)-2,3-Epoxyhexyl-p-toluolsulfonat in Form von hellgelben Kristallen, die aus Diethylether/n-Hexan als farblose Nadeln kristallisierten (Ausbeute: 71 %; [α]_{D}²⁰ = +36,3 (c = 2,0 in Trichlormethan)).

Die Bestimmung der optischen Reinheit erfolgte mittels chiraler Gaschromatographie. Dabei wurden die Enantiomeren (2S,3S)-2,3-Epoxyhexyl-p-toluolsulfonat und (2R,3R)-2,3-Epoxyhexyl-p-toluolsulfonat an einer 20 m langen Quarzglas-Säule mit 0,25 mm innerem Durchmesser, welche mit Heptakis-[2,3-di-O-methyl-6-O-({1,1,2-trimethylpropyl}-dimethyl)]-β-cyclodextrin beschichtet war, bei 155°C aufgetrennt. Das (2S,3S)-Isomer hatte einen Rₜ-Wert (Retentionszeit) von 32,82, sein Enantiomeres von 34,12 Minuten. Nach Umkristallisation lag der ee-Wert des (2R,3R)-Isomeren bei ca. 99 %.

### c) (2S,3R)-1-Brom-2,3-epoxyhexan

Eine Lösung von (2R,3R)-2,3-Epoxyhexyl-p-toluolsulfonat (13 g, 48,1 mmol) und Lithiumbromid (16 g, 0,186 mmol) in trockenem Aceton (200 ml) wurde 30 Minuten am Rückfluß gekocht. Nach Entfernen des Lösungsmittels wurden 200 ml Wasser und 100 ml Diethylether zugesetzt. Die organische Phase wurde abgetrennt und die Wasserphase zweimal mit jeweils 50 ml Diethylether extrahiert. Die vereinigten organischen Phasen wurden mit gesättigter Natriumhydrogencarbonat-Lösung und konzentrierter Kochsalzlösung gewaschen. Nach dem Trocknen über wasserfreiem Natriumsulfat wurde das Lösungsmittel entfernt. Der Rückstand wurde an 400 g Kieselgel chromatographiert (Laufmittel: 20 Vol.-% Ethylacetat in n-Hexan). Die Ausbeute betrug 96,4 % (farbloses Öl; [α]_{D}¹⁹ = +12,5 (c = 2,8 in Trichlormethan)).

### d) (3R)-1-Hexen-3-ol

Eine Mischung aus (2S,3R)-1-Brom-2,3-epoxyhexan (5,42 g, 30,2 mmol), Natriumiodid (12 g, 80 mmol) und Zinkstaub (5,9 g, 90 mmol) in 60 ml trockenem Methanol wurde 4 Stunden unter Argon am Rückfluß gekocht und nach dem Abkühlen in eine Mischung von 100 ml Diethylether, 100 ml n-Pentan und 200 ml Wasser ausgegossen. Nach Filtration wurde die organische Phase abgetrennt, und die wäßrige Phase wurde zweimal mit jeweils 50 ml Diethylether extrahiert. Die vereinigten organischen Phasen wurden mit konzentrierter Kochsalzlösung gewaschen. Der nach dem Trocknen über wasserfreiem Natriumsulfat und dem Einengen bei 40°/200 hPa erhaltene Rückstand wurde destilliert. (3R)-1-Hexen-3-ol wurde als farbloses Öl mit 74,7 % Ausbeute erhalten (Kp.: 70-72°C/120 hPa; [α]_{D}²⁰ = -14,7 (c = 1,9 in Trichlormethan)).

Die Bestimmung der optischen Reinheit erfolgte mittels chiraler Gaschromatographie. Dabei wurden die Enantiomeren (3R)-1-Hexen-3-ol und (3S)-1-Hexen-3-ol an einer 24 m langen Quarzglas-Säule mit 0,25 mm innerem Durchmesser, welche mit Octakis-(6-O-methyl-2,3-di-O-pentyl)-γ-cyclodextrin beschichtet war, bei 45°C aufgetrennt. Das (3R)-Isomer hatte einen Rₜ-Wert von 19,8, sein Enantiomeres von 21,2 Minuten. Der ee-Wert des (3R)-Isomeren lag bei ca. 99 %.

### e) (3R)-3-(tert.-Butyldimethylsilyloxy)-1-hexen

3,8 g (24 mmol) tert.-Butyldimethylsilylchlorid wurden in 5 Portionen über 10 Minuten bei -10°C zu einer gerührten Lösung von 2,05 g (20,5 mmol) (3R)-1-Hexen-3-ol und 3,4 g (49 mmol) Imidazol in 50 ml trockenem Dimethylformamid gegeben. Nach 3 Stunden bei 0°C wurde die Reaktionsmischung in 200 ml gesättigte wäßrige Natriumhydrogencarbonat-Lösung ausgegossen. Die Mischung wurde dreimal mit jeweils 50 ml n-Hexan extrahiert. Die vereinigten organischen Phasen wurden mit Wasser und konzentrierter Kochsalzlösung gewaschen. Nach dem Trocknen über wasserfreiem Natriumsulfat und Entfernen des Lösungsmittel wurde der Rückstand an 300 g Kieselgel chromatographiert. Als Laufmittel diente ein Gemisch von 5 Vol.-% Ethylacetat in n-Hexan. (3R)-3-(tert.-Butyldimethylsilyloxy)-1-hexen wurde als farbloses Öl mit 91,6 % Ausbeute erhalten ([α]_{D}¹⁹ = -5,3 (c = 2,4 in Trichlormethan)).

### f) (3R)-3-(tert.-Butyldimethylsilyloxy)-hexan-2-on

Eine Mischung von 0,39 g Palladium-(II)-chlorid (0,22 mmol) und 2,2 g Kupfer-(I)-chlorid (22 mmol) in 10 ml Wasser und 70 ml absolutem Dimethylformamid wurde 1 Stunde lang bei 60°C in einer Sauerstoffatmosphäre kräftig gerührt. Anschließend wurden 3,63 g (16,9 mmol) (3R)-3-(tert.-Butyldimethylsilyloxy)-1-hexen zugegeben. Die so erhaltene Mischung wurde 30 Minuten bei 60°C gerührt. Nach Zugabe von 300 ml Wasser und 100 ml n-Hexan wurde die organische Phase abgetrennt und die wäßrige Phase zweimal mit jeweils 50 ml n-Hexan extrahiert. Die vereinigten organischen Phasen wurden nacheinander mit einer gesättigten wäßrigen Natriumhydrogencarbonat-Lösung und konzentrierter Kochsalzlösung gewaschen. Nach dem Trocknen über wasserfreiem Natriumsulfat wurde eingeengt, und der Rückstand wurde an 300 g Kieselgel mit 10 Vol.-% tert.-Butylmethylether in n-Hexan als Laufmittel chromatographiert. (3R)-3-(tert.-Butyldimethylsilyloxy)-hexan-2-on wurde in einer Ausbeute von 65,1 % als farbloses Öl erhalten ([α]_{D}²⁰ = +33,7 (c = 2,1 in Trichlormethan))

### g) (3R)-3-Hydroxyhexan-2-on

1 ml 40 gew.-%iger Fluorwasserstoffsäure wurde zu einer Lösung von 2,3 g (10 mmol) (3R)-3-(tert.-Butyldimethylsilyloxy)-hexan-2-on in 15 ml Acetonitril gegeben. Nachdem die Reaktionsmischung 3 Stunden bei 20°C gerührt worden war, wurde sie in 100 ml einer gesättigten wäßrigen Natriumhydrogencarbonat-Lösung ausgegossen. Die Mischung wurde dreimal mit jeweils 50 ml Diethylether extrahiert. Die vereinigten Diethylether-Extrakte wurden mit konzentrierter Kochsalzlösung gewaschen und über wasserfreiem Natriumsulfat getrocknet. Nach dem Einengen des Lösungsmittels blieb ein gelbes Öl zurück, welches an 100 g Kieselgel mit 20-40 Vol.-% Diethylether in n-Pentan als Laufmittel chromatographiert wurde. (3R)-3-Hydroxy-hexan-2-on wurde als farbloses Öl in einer Ausbeute von 90 % erhalten ([α]_{D}²⁰ = -110 (c = 0,8 in Trichlormethan)).

Die Bestimmung der optischen Reinheit erfolgte mittels chiraler Gaschromatographie. Dabei wurden die Enantiomeren (3R)-3-Hydroxyhexan-2-on und (3S)-3-Hydroxy-hexan-2-on an einer 25 m langen Quarzglas-Säule mit 0,25 mm innerem Durchmesser, welche mit Heptakis-(2,6-di-O-methyl-3-O-pentyl)-β-cyclodextrin beschichtet war, bei 70°C aufgetrennt. Das (3R)-Isomer hatte einen Rₜ-Wert von 4,16, sein Enantiomeres von 6,02 Minuten. Der ee-Wert des (3R)-Isomeren lag bei ca. 99 %.

### Beispiel 2

### Herstellung von (2S,3R)-Hexan-2,3-diol

### a) (2S,3R)-1,2-Epoxy-3-hexanol

Gepulvertes aktiviertes Molekularsieb (4 Å, 10 g), 4,55 g (16 mmol) Titan-(IV)-iso-propylat und 18,1 g (200 mmol) tert.-Butylhydroperoxid wurden nacheinander einer Lösung von Di-iso-propyl-D-(-)-tartrat (4,48 g, 20 mmol) in trockenem Dichlormethan (200 ml) bei -30°C unter Argon zugesetzt. Die Mischung wurde noch 20 Minuten bei -30°C gerührt. Danach wurden innerhalb von 15 Minuten 9 g (90 mmol) frisch destilliertes 1-Hexen-3-ol in 50 ml trockenem Dichlormethan zugefügt. Die Mischung wurde noch 48 Stunden bei -30°C gerührt. Anschließend wurde sie langsam in eine eiskalte Lösung von 30 g (0,2 mol) Weinsäure und 33 g (0,12 mol) Eisen-(II)-sulfat-Heptahydrat in 200 ml Wasser ausgegossen. Die so entstandene Mischung wurde 20 Minuten lang kräftig gerührt. Die organische Phase wurde abgetrennt, und die Wasserphase wurde zweimal mit jeweils 50 ml Dichlormethan extrahiert. Die vereinigten organischen Phasen wurden mit Wasser und konzentrierter Kochsalzlösung gewaschen, über wasserfreiem Natriumsulfat getrocknet, und man entfernte danach das Lösungsmittel. Es folgte eine Chromatographie des Rückstands an 700 g Kieselgel (25-50 Vol.-% Ethylacetat in n-Hexan). Die Ausbeute an (2S,3R)-1,2-Epoxy-3-hexanol betrug 79 % (farbloses Öl; [α]_{D}²⁰ = -24,3 (c = 1,8 in Trichlormethan)).

Die Bestimmung der optischen Reinheit erfolgte mittels chiraler Gaschromatographie. Dabei wurden die Enantiomeren (2S,3R)-1,2-Epoxy-3-hexanol und (2R,3S)-1,2-Epoxy-3-hexanol an einer 25 m langen Quarzglas-Säule mit 0,25 mm innerem Durchmesser, welche mit Heptakis-(2,6-di-O-methyl-3-O-pentyl)-β-cyclodextrin beschichtet war, bei einem Temperaturprogramm (3 Minuten 60°C, danach mit 5°C/min bis 120°C) aufgetrennt. Das (2R,3S)-Isomere hatte einen Rₜ-Wert von 7,95, sein Enantiomeres von 8,22 Minuten. Der ee-Wert des (2S,3R)-Isomeren lag bei ca. 97 %.

### b) (2S,3R)-1,2-Epoxy-3-(2-methoxy-2-methylethoxy)-hexan

10 mg p-Toluolsulfonsäure wurden bei 0°C zu 2,9 g (25 mmol) (2S,3R)-1,2-Epoxy-3-hexanol in 8 g (0,11 mmol) 2-Methoxypropen gegeben. Nach 30-minütigem Rühren bei 20°C wurden 30 mg Kaliumcarbonat hinzugefügt. Die Mischung wurde im Vakuum eingeengt, und danach wurde der Rückstand an 300 g Kieselgel chromatographiert. Als Laufmittel diente n-Pentan mit 20-30 Vol.-% Diethylether und 0,2 Vol.-% Triethylamin. Die Ausbeute an (2S,3R)-1,2-Epoxy-3-(2-methoxy-2-methylethoxy)-hexan, einem farblosen Öl, betrug 83% ([α]_{D}²⁰ = -32,5 (c = 2,0 in Dichlormethan))

### c) (2S,3R)-3-(2-Methoxy-2-methylethoxy)-hexan-2-ol

3,62 g (19,2 mmol) (2S,3R)-1,2-Epoxy-3-(2-methoxy-2-methylethoxy)-hexan in 30 ml Diethylether wurden unter Argon über 15 Minuten bei 0°C zu einer gerührten Suspension von 0,85 g (22,2 mmol) Lithiumaluminiumhydrid in 100 ml Diethylether getropft. Es wurde 36 Stunden bei 20°C nachgerührt. Durch vorsichtige Zugabe von 1,2 ml Wasser wurde sodann der Überschuß an Lithiumaluminiumhydrid zersetzt. Nach 5 Minuten wurden 4 ml einer 10 gew-%igen Natronlauge zugegeben und 10 Minuten kräftig gerührt. Danach wurde der Niederschlag abfiltriert, und der Filterrückstand wurde dreimal mit 50 ml Tetrahydrofuran extrahiert. Die vereinigten organischen Phasen wurden über wasserfreiem Natriumsulfat getrocknet und eingeengt. Der Rückstand wurde über Kaliumcarbonat destilliert und lieferte (2S,3R)-3-(2-Methoxy-2-methylethoxy)-hexan-2-ol in einer Ausbeute von 83 % (Kp.: 71 - 73°C/3 hPa; [α]_{D}¹⁹ = -10,3 (c = 2,3 in Dichlormethan)).

### d) (2S,3R)Hexan-2,3-diol

Zu einer Lösung von 2,7 g (14,2 mmol) (2S,3R)-3-(2-Methoxy-2-methylethoxy)-hexan-2-ol in 10 ml Methanol wurden 5 ml 1 N Salzsäure gegeben. Die Mischung wurde 30 Minuten bei 20°C gerührt und danach in eine Lösung von 5 g Kaliumcarbonat in 10 ml Wasser ausgegossen. 50 ml konzentrierter Kochsalzlösung wurden zugesetzt und die Mischung dreimal mit jeweils 50 ml Ethylacetat extrahiert. Die vereinigten organischen Phasen wurden über wasserfreiem Natriumsulfat getrocknet und danach eingeengt. Der Rückstand wurde an 100 g Kieselgel mit 40-70 Vol.-% Ethylacetat in n-Hexan als Laufmittel chromatographiert. (2S,3R)-Hexan-2,3-diol wurde nach Destillation als farbloses Öl mit einer Ausbeute von 86,4 % erhalten (Kp.: 103°C/19 hPa; Kristallisation nach 30 Minuten bei 20°C; [α]_{D}²⁰ = 20,9 (c = 1,4 in Trichlormethan)).

Die Bestimmung der optischen Reinheit erfolgte mittels chiraler Gaschromatographie. Dabei wurden die 4 Stereoisomeren an einer 25 m langen Quarzglas-Säule mit 0,25 mm innerem Durchmesser, welche mit Heptakis-(2,6-di-O-methyl-3-O-pentyl)-β-cyclodextrin beschichtet war, bei 85°C aufgetrennt. Rₜ-Werte: (2S,3S)-Isomer 12,3, (2R,3R)-Isomer 14, (2R,3S)-Isomer 15,4 und (2S,3R)-Isomer 16,9 Minuten. Der ee-Wert und der de-Wert des (2S,3R)-Isomeren lagen bei ca. 96 %.

### Beispiel 3

### Herstellung von (2R,3R)-Hexan-2,3-diol

### a) (4R,5R)-[5-(Hydroxymethyl)-2,2-dimethyl-1,3-dioxolan-4-yl]-methyl-p-toluolsulfonat

21,7 mmol n-Butyllithium (1,59 molar in n-Hexan, 13,6 ml) wurden bei -78°C zu einer Lösung von (4R,5R)-4,5-Bis-(hydroxymethyl)-2,2-dimethyl-1,3-dioxolan (3,32 g, 20,5 mmol) in 60 ml Tetrahydrofuran gegeben ((4R,5R)-4,5-Bis-(hydroxymethyl)-2,2-dimethyl-1,3-dioxolan wurde hergestellt gemäß J. Org. Chem. 33, Seite 2171 (1968)). Nach 10-minütigem Rühren wurde ihr eine auf -78°C gekühlte Lösung von p-Toluolsulfonylchlorid (4 g, 21 mmol) in 20 ml Tetrahydrofuran zugesetzt. Nach 15-minütigem Rühren bei -78°C wurde die Mischung auf 20°C erwärmt und in 200 ml konzentrierte Kochsalzlösung ausgegossen. Die organische Phase wurde abgetrennt und die wäßrige Phase zweimal mit 50 ml Ethylacetat extrahiert. Die vereinigten organischen Phasen wurden über wasserfreiem Natriumsulfat getrocknet und danach eingeengt. Der Rückstand wurde an 400 g Kieselgel mit Ethylacetat und n-Hexan im Gewichtsverhältnis 60:40 als Laufmittel chromatographiert. Es wurden 74,6 % (4R,5R)-[5-(Hydroxymethyl)-2,2-dimethyl-1,3-dioxolan-4-yl]-methyl-p-toluolsulfonat erhalten ([α]_{D}¹⁹ = +11,8 (c = 1,88 in Trichlormethan); vgl. J. Org. Chem. 55, Seite 4417 (1990): [α]_{D}²¹ = +11,3 (c = 2,70 in Trichlormethan)).

### b) (4R,5R)-(2,2-Dimethyl-5-propyl-1,3-dioxolan-4-yl)-methanol

3,29 g (16 mmol) Kupfer-(I)-bromid/Dimethylsulfid und ein Grignard-Reagenz, hergestellt aus 3,92 g (36 mmol) Ethylbromid und 1,3 g (54 mmol) Magnesium in 60 ml Diethylether, wurden nacheinander zu einer gerührten Lösung von (4R,5R)-[5-(Hydroxymethyl)-2,2-dimethyl-1,3-dioxolan-4-yl]-methyl-p-toluolsulfonat (4,62 g, 14,6 mmol) in 60 ml Tetrahydrofuran bei -40°C unter Argon gegeben. Nach 4-stündigem Rühren bei 0°C wurde die Mischung in ein Gemisch aus 200 ml gesättigter wäßriger Ammoniumchlorid-Lösung und 10 ml gesättigter wäßriger Ammoniak-Lösung gegossen. Das entstehende 2-Phasen-System wurde 20 Minuten gerührt. Danach wurde die organische Phase abgetrennt und die wäßrige Phase mit Ethylacetat extrahiert. Die vereinigten organischen Phasen wurden mit gesättigter wäßriger Ammoniumchlorid-, Natriumhydrogencarbonat- und konzentrierter Kochsalzlösung gewaschen. Nach dem Trocknen über wasserfreiem Natriumsulfat und Entfernen des Lösungsmittels blieb ein Rückstand, der an 100 g Kieselgel mit Ethylacetat und n-Hexan im Gewichtsverhältnis 1:3 als Laufmittel chromatographiert wurde. (4R,5R)-(2,2-Dimethyl-5-propyl-1,3-dioxolan-4-yl)-methanol wurde als farbloses Öl mit einer Ausbeute von 83,3 % erhalten ([α]_{D}²⁰ = +27,3 (c = 2,81 in Trichlormethan); vgl. Liebigs Ann. Chem. Seite 2261 (1975): [α]_{D}²⁵ = -27,7 (c = 2,8 in Trichlormethan) für das Enantiomer).

### c) (4R,5R)-(2,2-Dimethyl-5-propyl-1,3-dioxolan-4-yl)-methanol-p-toluolsulfonat

2,29 g (12 mmol) p-Toluolsulfonylchlorid wurden bei 0°C zu einer gerührten Mischung von 4,6 g (80 mmol) gepulvertem Kaliumhydroxid und 1,94 g (11,1 mmol) (4R,5R)-(2,2-Dimethyl-5-propyl-1,3-dioxolan-4-yl)-methanol in 100 ml Diethylether gegeben. Nach 5-stündigem Rühren bei 0°C wurde die Mischung in 100 ml Wasser gegossen. Die organische Schicht wurde abgetrennt und die wäßrige Phase zweimal mit 50 ml Diethylether extrahiert. Die vereinigten organischen Phasen wurden mit einer gesättigten wäßrigen Natriumhydrogencarbonat- und konzentrierter Kochsalzlösung gewaschen, über wasserfreiem Natriumsulfat getrocknet und danach eingeengt. Der Rückstand wurde an 300 g Kieselgel mit n-Hexan, welchem 15-30 Vol.-% Ethylacetat beigemischt waren, chromatographiert. (4R,5R)-(2,2-Dimethyl-5-propyl-1,3-dioxolan-4-yl)-methanol-p-toluolsulfonat wurde als farbloses Öl in einer Ausbeute von 91 % erhalten ([α]_{D}²⁰ = 21,2 (c = 1,7 in Trichlormethan)).

### d) (4R,5R)-2,2,5-Trimethyl-4-propyl-1,3-dioxolan

Eine Lösung von (4R,5R)-(2,2-Dimethyl-5-propyl-1,3-dioxolan-4-yl)-methanol-p-toluolsulfonat (3,04 g, 9,25 mmol) in 30 ml Diethylether wurde bei 0°C und unter Argon zu einer gerührten Suspension von Lithiumaluminiumhydrid (0,380 g, 10 mmol) in 50 ml Diethylether getropft. Es wurde noch 24 Stunden bei 20°C gerührt. Der Überschuß an Lithiumaluminiumhydrid wurde durch Zugabe von 0,2 ml Wasser zerstört. Nach 5-minütigem Rühren wurde die Mischung mit 1,8 ml 10 gew.-%iger Natronlauge behandelt. Die resultierende Mischung wurde noch weitere 10 Minuten kräftig gerührt. Dann wurde der Niederschlag abfiltriert und der Filterrückstand dreimal mit jeweils 50 ml Tetrahydrofuran extrahiert. Die vereinigten organischen Phasen wurden über wasserfreiem Natriumsulfat getrocknet. Der Rückstand lieferte nach Chromatographie an 100 g Kieselgel unter Verwendung von n-Pentan mit 10-20 Vol.-% Diethylether als Laufmittel 92 % (4R,5R)-2,2,5-Trimethyl-4-propyl-1,3-dioxolan als farbloses Öl ([α]_{D}²⁰ = +6,28 (c = 2,5 in Trichlormethan)).

### e) (2R,3R)-Hexan-2,3-diol

3 ml 6 N Salzsäure wurden zu einer Lösung von 1,13 g (7,14 mmol) (4R,5R)-2,2,5-Trimethyl-4-propyl-1,3-dioxolan in 5 ml Methanol und 3 ml Tetrahydrofuran bei 0°C gegeben. Die Mischung wurde 60 Minuten bei 20°C gerührt und dann in eine Lösung von 5 g Kaliumcarbonat in 20 ml Wasser gegossen. 50 ml konzentrierte Kochsalzlösung wurden zugegeben und die resultierende Mischung dreimal mit jeweils 50 ml Ethylacetat extrahiert. Die vereinigten organischen Phasen wurden über wasserfreiem Natriumsulfat getrocknet, und danach wurden sie eingeengt. Der Rückstand wurde an 60 g Kieselgel mit 40-70 Vol.-% Ethylacetat in n-Hexan chromatographiert und lieferte nach Destillation (2R,3R)-Hexan-2,3-diol als farbloses Öl in einer Ausbeute von 81 % (Kp.: 102°C/17 hPa; [α]_{D}²⁰ = 22,3 (c = 1,22 in Trichlormethan)).

Die Bestimmung der optischen Reinheit erfolgte mittels chiraler Gaschromatographie. Dabei wurden die 4 Stereoisomeren an einer 25 m langen Quarzglas-Säule mit 0,25 mm innerem Durchmesser, welche mit Heptakis-(2,6-di-O-methyl-3-O-pentyl)-β-cyclodextrin beschichtet war, bei 85°C aufgetrennt. Rₜ-Werte: (2S,3S)-Isomer 12,3, (2R,3R)-Isomer 14, (2R,3S)-Isomer 15,4 und (2S,3R)-Isomer 16,9 Minuten. Der ee-Wert und der de-Wert des (2S,3R)-Isomeren lagen bei > 99,5 %.

### Beispiel 4

### Herstellung von racemischem 3-Hydroxyhexan-2-on

### a) 2-(1-Hydroxybutyl)-2-methyl-1,3-dithian

56 mmol n-Butyllithium (35 ml einer 1,6 M Lösung in Hexan) wurden unter Rühren zu einer Lösung von 2-Methyl-1,3-dithian (7 g, 52,1 mmol) in 100 ml trockenem Tetrahydrofuran bei -30°C unter Argon gegeben. Nach zweistündigem Rühren bei -30°C wurde die Mischung auf -78°C gekühlt. Es wurden 4,2 g (58,3 mmol) frisch destillierter n-Butyraldehyd innerhalb von 15 Minuten zugetropft und die Mischung noch 30 Minuten nachgerührt. Nach dem Erwärmen auf 20°C wurde sie in ein Becherglas mit 300 ml Wasser und 100 ml n-Hexan ausgegossen. Die organische Phase wurde abgetrennt, die Wasserphase dreimal mit jeweils 50 ml Dichlormethan extrahiert. Die vereinigten organischen Phasen wurden mit 10 gew.-%iger Kalilauge, Wasser und konzentrierter Kochsalzlösung gewaschen, über wasserfreiem Natriumsulfat getrocknet und danach destilliert. Es wurden 8,82 g 2-(1-Hydroxybutyl)-2-methyl-1,3-dithian als farbloses Öl isoliert.

### b) 3-Hydroxyhexan-2-on

6 g (29,1 mmol) 2-(1-Hydroxybutyl)-2-methyl-1,3-dithian wurden in 70 ml Aceton und 10 ml Wasser vorgelegt. Die Mischung wurde auf -10°C gekühlt. Es wurden bei dieser Temperatur 13,5 g (60 mmol) N-Iodsuccinimid zugegeben und das Ganze noch 1 Stunde bei dieser Temperatur nachgerührt. Danach wurde die Mischung in 100 ml gesättigte Natriumthiosulfat-Lösung ausgegossen, das Ganze dreimal mit jeweils 50 ml Ethylacetat extrahiert, die vereinigten organischen Phasen mit gesättigter Natriumthiosulfat-Lösung, Wasser und konzentrierter Kochsalzlösung gewaschen, über wasserfreiem Natriumsulfat getrocknet und eingeengt. Säulenchromatographie an 300 g Kieselgel mit 15-40 Gew.-% Ethylacetat in n-Hexan als Laufmittel lieferte 2,21 g der Titelverbindung als farbloses Öl.

### Beispiel 5

### Herstellung von racemischem 2-Hydroxyhexan-3-on

### a) 2-(1-Hydroxyethyl)-2-n-propyl-1,3-dithian

Die Durchführung der Synthese erfolgte analog zu Beispiel 4/(a), jedoch mit 8 g (49,3 mmol) 2-n-Propyl-1,3-dithian, 52 mmol n-Butyllithium (32,5 ml einer 1,6 M Lösung in n-Hexan) und 2,5 g (56,8 mmol) Acetaldehyd. Es wurden 8,84 g 2-(1-Hydroxyethyl)-2-n-propyl-1,3-dithian als farbloses Öl erhalten.

### b) 2-Hydroxyhexan-3-on

Die Spaltung des 2-(1-Hydroxyethyl)-2-n-propyl-1,3-dithian zum 2-Hydroxyhexan-3-on erfolgte analog zu Beispiel 4/(b), jedoch mit 1,5 g (7,3 mmol) 2-Hydroxyhexan-3-on und 3,37 g (15 mmol) N-Iodsuccinimid. Säulenchromatographie an 60 g Kieselgel mit 15-30 Gew.-% Ethylacetat in n-Hexan als Laufmittel erbrachten 0,48 g der Titelverbindung als farbloses Öl.

### Beispiel 6

### Herstellung von racemischem Hexan-2,3-diol

Zu einer Suspension von 750 mg (20 mmol) Lithiumaluminiumhydrid in 50 ml absolutem Diethylether wurden bei 0°C 1,2 g (10 mmol) racemisches 2-Hydroxyhexan-3-on in 30 ml Diethylether getropft. Es wurde 1 Stunde bei 0°C nachgerührt. Anschließend wurden 0,2 ml Wasser zugegeben. Nach Zugabe von 2 ml 10 gew.-%iger Natronlauge wurde noch 10 Minuten gerührt. Der Niederschlag wurde abfiltiriert und der Filterkuchen noch zweimal mit jeweils 30 ml Tetrahydrofuran gewaschen. Die vereinigten organischen Phasen wurden über wasserfreiem Natriumsulfat getrocknet und anschließend eingeengt. Nach Destillation im Vakuum wurden 0,92 g eines Gemisches aus (2S*,3R*)-Hexan-2,3-diol und (2R*,3R*)-Hexan-2,3-diol im Verhältnis 3:1 erhalten (Kp: 100°C/17 hPa).

Die Schreibweise z.B. "(2S*,3R*)" bedeutet ein Verhältnis von (2S,3R) zu (2R,3S) von 1:1, also ein Racemat der reinen Diastereomeren.

### Beispiel 7

### Herstellung einer erfindungsgemäßen Mischung

500 mg eines Gemisches aus (2S*,3R*)-Hexan-2,3-diol und (2R*,3R*)-Hexan-2,3-diol im Verhältnis 77:23 sowie 230 mg (2S,3R)-Hexan-2,3-diol vom ee-Wert 96 % und 55 mg (2S,3R)-Hexan-2,3-diol vom ee-Wert 99 % wurden bei 80°C 30 Minuten verrührt. Man erhielt 785 mg eines Gemisches aus (2S,3R)-Hexan-2,3-diol mit 34 % ee und (2R,3R)-Hexan-2,3-diol mit 32 % ee im Verhältnis 78,5:21,5.

Durch Zugabe von 200 mg (3R)-3-Hydroxyhexan-2-on (ee-Wert: 99 %, vgl. Beispiel 1) zu 20 mg des Diolgemischs gemäß Beispiel 7 wurde eine Mischung erhalten, welche die nachstehende Zusammensetzung aufwies:

90,9 Gew.-Teile (3R)-3-Hydroxyhexan-2-on (99 % ee), 7,1 Gew.-Teile (2S,3R)-Hexan-2,3-diol (34 % ee), 2 Gewichtsteile (2R,3R)-Hexan-2,3-diol (32 % ee).

### Beispiel 8

### Versuch im Windkanal mit Hylotrupes bajulus

Die Testkäfer stammten aus einer Laborzucht des Hylotrupes bajulus. Vor Versuchseinsatz wurden die Käfer, nachdem sie aus ihren Brutholzblöcken erschienen waren, für 8 Tage einzeln in Plastikboxen mit angefeuchtetem Filterpapier gehalten, um Geschlechtsreife und Kopulationsbereitschaft der Käfer voraussetzen zu können. Die Käfer mußten nur mit Trinkwasser versorgt werden. Männchen und Weibchen wurden getrennt nach Geschlecht in 2 Klimakammern mit einer konstanten Temperatur von 20°C und 12:12 L:D Photoperiode gehalten. Die Käfer, die bis zum Einsatz im Windkanalversuch keinen Kontakt mit Artgenossen hatten, wurden innerhalb von 7 Tagen in jedem der Versuchsansätze einmal getestet. Während des Versuchszeitraums ergab sich ein Alter von 9-15 Tage für die kopulationsbereiten Versuchstiere. Es wurden Weibchen vom 3.-19. Tag nach Erscheinen oder Schlupf aus ihrem Brutholz bei der Kopulation beobachtet.

Nach Beendigung der Versuchsansätze im Windkanal mit den virginen Weibchen wurden sie mit jeweils einem Männchen zusammengesetzt und sofort nach der Kopulation wieder getrennt. Die kopulierten Weibchen wurden 24 Stunden nach der Kopulation erneut im Windkanal getestet.

Die Versuchsdurchläufe im Windtunnel (s. Figur) wurden in einer Klimakammer bei 30°C und einer relativen Luftfeuchtigkeit von 20-30 % durchgeführt. Durch Fluoreszenzlicht von 8 Neonröhren (1) über dem Windkanal wurde der Versuchsaufbau von oben gleichmäßig und konstant ausgeleuchtet (8000 Lux gemessen im Windkanalrohr). Der Windtunnel bestand aus einer Acryl-Plexiglasröhre (2) mit 100 cm Länge und 19 cm Innendurchmesser, die durch Markierungen in 4 Zonen (I.-IV.) zu jeweils 25 cm unterteilt war. Das Windkanalrohr wurde von verschiebbaren Metallträgern entlang einer Schienen (3) gehalten. Die beiden Röhrenöffnungen wurden an der einen Seite durch ein Startgitter (4) und auf der gegenüberliegenden Seite durch ein Gitter (5) direkt vor dem Ventilator (6) verschlossen. Die Versuchstiere wurden auf dem Startgitter eingesetzt, während die Stimulantien gleichzeitig in einem Gaze-Behältnis (7 = Petrischale mit Gazedeckel und -boden; 14 cm Durchmesser) an das Ventilatorgitter geheftet wurden. Die Windseite des Ventilators wurde mit mehreren Lagen aus Gaze überspannt, um eine gleichmäßige und laminare Strömung durch die Windkanalzonen zu erhalten. Die kontaminierte Luft, die durch das Startgitter ausströmte, wurde über einen Glastrichter (8) durch eine Absaugvorrichtung (9) aus der Klimakammer in die Entlüftung geleitet.

Die Windgeschwindigkeiten im Rohr betrugen durchschnittlich am Startgitter 0,65 m/sec, in der Mitte des Rohres zwischen Zone II und III 0,71 m/sec und am Ventilatorgitter neben dem Gaze-Behältnis 2,3 m/sec. Direkt vor dem Gaze-Behältnis wurde die Windgeschwindigkeit auf 0,02 m/sec reduziert, da der Wind die 2 Gaze-Lagen von Schalendeckel und -boden passieren mußte.

Die Versuchsdurchläufe wurden zwischen 12.00 und 17.00 Uhr durchgeführt, um den Zeitrahmen der Tagesaktivität der Käfer auszunutzen. In jedem Testlauf wurden 2 Versuchskäfer gleichzeitig am Startgitter eingesetzt und für 15 Minuten Versuchsdauer beobachtet. Als Stimulantien wurden entweder lebende Käfer oder Glaskapillare, die mit Hexanlösungen von männlichen CLSA-Filtraten oder synthetischen Drüsensekretkomponenten gefüllt waren, im Gaze-Behältnis eingeschlossen. Die Versuchsdauer und die Kapillarengrößen waren so ausgewählt worden, daß gegen Ende der 15 Minuten Versuchsdauer eine komplette Verdunstung des Hexanvolumens garantiert war. Alle 5 µl Hexanlösung wurden in geeichten 5 µl Mikropipetten angeboten, die für die Versuchsdurchläufe im Windkanal im Gaze-Behältnis waagerecht befestigt wurden.

Folgende Versuchsansätze wurden im Einzelnen getestet:
1) Kontrolle mit einem leeren Gaze-Behältnis;
2) Hexan-Kontrolle mit 5 µl n-Hexan;
3) unbegattete Weibchen, die durch Kartonstreifen in der Gazebox getrennt waren und keinen Sichtkontakt hatten;
4) unbegattete Männchen, ebenfalls durch Kartonstreifen isoliert;
5) CLSA-Extrakt: Die Gewinnung eines Pheromonextraktes erfolgte mittels der Closed Loop Stripping Analysis (CLSA; vgl. Literaturzitate in der Beschreibung). In einem 110 ml Glasgefäß wurden 8 unbegattete Männchen auf einigen Filterpapierstreifen eingesetzt. Die Papierstreifen dienten als Isolierung und Sitzplatz für die Käfer im engen Gefäß. Für 24 Stunden unter konstanten Lichtbedingungen (von 8 Neonröhren) wurde die Gefäßluft, die mit den Lockstoffen der Männchen angereichert war, mit einem 1,5 mg Aktivkohle-Filter filtriert. Der Filter wurde mit 15 µl n-Hexan extrahiert. Die Anreicherung des Pheromones im Hexanextrakt wurde überprüft, indem 1 µl Hexangemisch im GC/MS qalitativ analysiert wurde. Der CLSA-Extrakt wurde nur im Windkanalversuch verwendet, wenn das Pheromon im Lösungsmittel nachweisbar war. Für 2 Versuchsdurchläufe wurden jeweils 5 µl eines CLSA-Extraktes in den geeichten 5 µl-Kapillaren (s.o.) eingesetzt;
6) 5 µl synthetische Mischung: Für das Angebot einer synthetischen Lockstoffmischung im Windkanal wurden 50 mg (3R)-3-Hydroxyhexan-2-on (ee > 99 %), 15 mg der diastereomeren Diole (2R,3R)-Hexan-2,3-diol (31 % ee) und (2S,3R)-Hexan-2,3-diol (34 % ee) im Verhältnis 1:3 und 1,5 mg Hexan-2,3-dion (4) in 5 ml Hexan gelöst. Jeweils 5 µl der synthetischen Mischung wurden angeboten;
7) 50 µl synthetische Mischung: Die höhere Dosierung des Lockstoffangebotes wurde durch die komplette Verdunstung von 50 µl der synthetischen Lösung (s. Ansatz 6) innerhalb von 15 Minuten erreicht, indem zwei 100 µl-Mikropipetten jeweils mit 25 µl der Pheromonlösung gefüllt wurden;
8) (3R)-3-Hydroxyhexan-2-on: 50 µg der synthetischen Hauptkomponente (ee > 99 %) waren in 5 µl Hexan enthalten;
9) (2R,3R)-Hexan-2,3-diol und (2S,3R)-Hexan-2,3-diol: 50 µg der Mischung der diastereomeren Diole wurde im Verhältnis 1 (31 % ee) : 3 (34 % ee) in 5 µl Hexan gelöst;
10) Hexan-2,3-dion: 50 µg wurden in 5 µl Hexan angeboten.

Folgende Verhaltensweisen der Käfer wurden registriert: Testkäfer beginnen auf dem Startgitter zu laufen und erreichen die Zonen I-IV; Suchbewegung auf dem Gaze-Behältnis/Einhalt der Bewegungen bei der Geruchsquelle auf dem Ventilatorgitter bzw. dem Gaze-Behältnis. Die Anzahl der Testkäfer, die die festgelegten Markierungen erreichten oder die definierten Verhaltensweisen zeigten, wurden durch den χ²-Test statistisch ausgewertet.

Die Ergebnisse der Versuche sind der folgenden Tabelle 1 zu entnehmen.

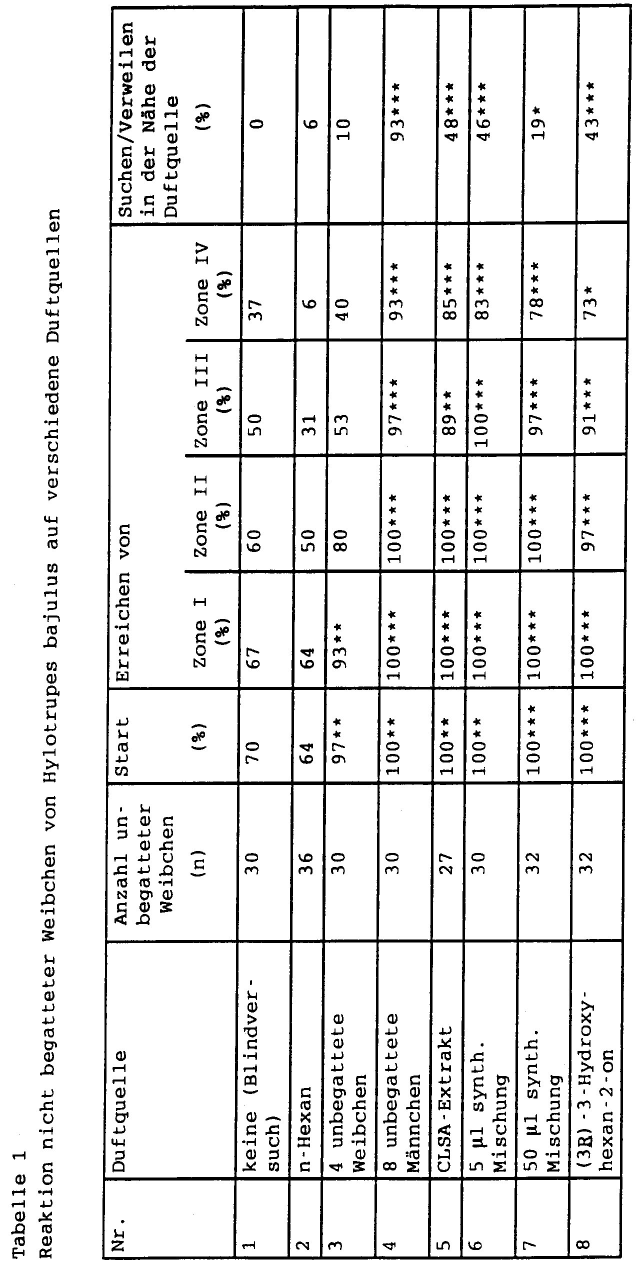

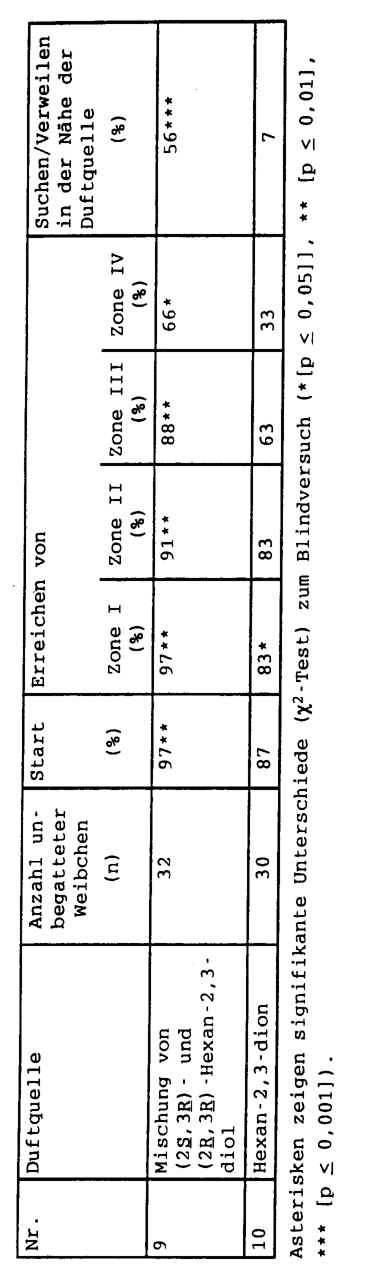

### Beispiel 9

### Versuch im Windkanal mit Pyrrhidium sanguineum

Die Tiere stammten aus eingetragenem Eichenholz. Die adulten Käfer wurden sofort nach ihrem Erscheinen aus dem Brutholz in Gläschen vereinzelt. Die Versuchstiere wurden bereits 4-7 Tage nach ihrem Erscheinen aus den Eichenästen eingesetzt.

Die Haltung der Versuchstiere, der Aufbau des Windkanals sowie die Versuchsbedingungen wurden für den Vergleich wie im Windkanalversuch von Hylotrupes bajulus gehalten (vgl. Beispiel 8). Es wurde eine Versuchstemperatur von 22°C gewählt. Die Windgeschwindigkeit im Windkanalrohr wurde durch zusätzliche Gaze-Lagen vor der Windseite des Ventilators gedrosselt. Sie betrug am Startgitter 0,31 m/sec, in der Mitte der Röhre (an der Grenze zwischen Zone II und III) 0,44 m/sec und am Ventilatorgitter 0,94 m/sec. Die Windgeschwindigkeiten in den Randbereichen waren jeweils etwas höher als in der Mitte des Röhrenquerschnitts.

Die Ergebnisse der Versuche sind in der Tabelle 2 zusammengestellt.

Als Stimulantien wurden die Versuchskonstellationen 2./3./4./6./8./9./10. aus dem Windkanalversuch mit Hylotrupes bajulus (Beispiel 8) übernommen. Als Unterschied wurde im 3. Versuchsansatz die Zahl der unbegatteten Weibchen in dem Gaze-Behältnis auf 8 Individuen erhöht. Für den Ansatz 6 wurde ein Gemisch von 8 Gew.-% (2R,3R)-Hexan-2,3-diol (31 % ee) und 92 Gew.-% (2S,3R)-Hexan-2,3-diol (34 % ee) verwendet.

Die begatteten Weibchen wurden 24 h nach der Paarung wiederholt in den Versuchen mit Ansatz 1 und 4 eingesetzt. Da manche Weibchen zu diesem Zeitpunkt bereits ihre durchschnittliche Lebensdauer erreicht hatten, wurden die altersschwachen Weibchen (17-14 Individuen) aus dem Versuch genommen.

Die Auswertung des Verhaltens im Windkanal erfolgte wie bei Hylotrupes bajulus.

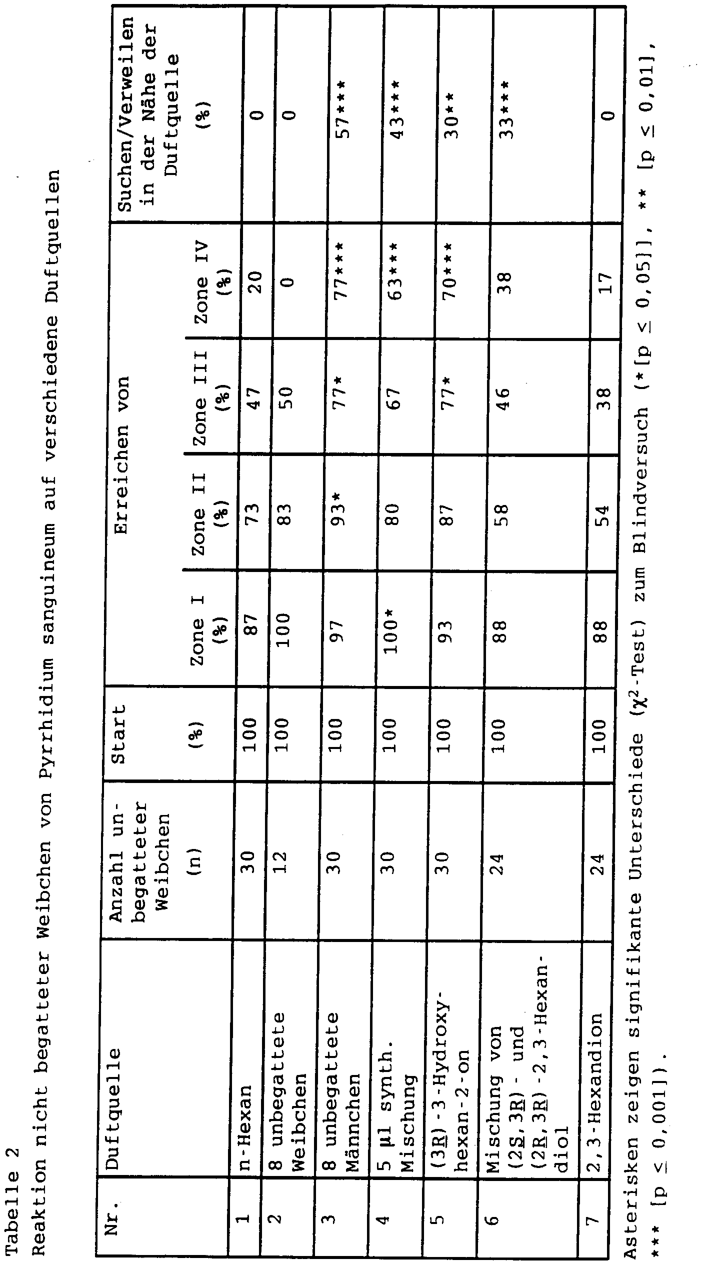

## Patentansprüche

1. Mischung, enthaltend ein Stereoisomeres von 3-Hydroxyhexan-2-on und zwei Stereoisomere von Hexan-2,3-diol.

2. Mischung nach Anspruch 1, dadurch gekennzeichnet, daß es sich bei dem Stereoisomeren von 3-Hydroxyhexan-2-on um (3R)-3-Hydroxyhexan-2-on handelt.

3. Mischung nach Anspruch 1 oder 2, dadurch gekennzeichnet daß es sich bei den Stereoisomeren von Hexan-2,3-diol um (2S,3R)-Hexan-2,3-diol und (2R,3R)-Hexan-2,3-diol handelt.

4. Mischung von (2S,3R)-Hexan-2,3-diol und (2R,3R)-Hexan-2,3-diol.

5. Mischung nach den Ansprüchen 1, 2 und 3, dadurch gekennzeichnet, daß sie
a) 70 bis 100 Gewichtsteile (3R)-3-Hydroxyhexan-2-on,
b) 0,01 bis 7 Gewichtsteile (2R,3R)-Hexan-2,3-diol,
c) 0,01 bis 20 Gewichtsteile (2S,3R)-Hexan-2,3-diol
und gewünschtenfalls
d) 0,01 bis 5 Gewichtsteile Hexan-2,3-dion und/oder
e) 0,01 bis 7 Gewichtsteile eines racemischen Gemisches von 2-Hydroxyhexan-3-on
enthält.

6. Zur Bekämpfung von Hylotrupes bajulus und/oder Pyrrhidium sanguineum nach der Paarungsstörungsmethode geeignetes Mittel, enthaltend eine wirksame Menge einer Mischung gemäß den Ansprüchen 1 bis 3 oder eine Mischung zweier beliebiger Stereoisomeren von Hexan-2,3-diol und einen flüssigen oder festen Trägerstoff.

7. Zur Bekämpfung von Hylotrupes bajulus und/oder Pyrrhidium sanguineum nach der Monitor-, Abfang- oder Paarungsstörungsmethode geeignetes Mittel, enthaltend eine wirksame Menge an (3R)-3-Hydroxyhexan-2-on oder einer Mischung gemäß Anspruch 4 oder 5 und einen flüssigen oder festen Trägerstoff.

8. Verwendung einer Mischung gemäß den Ansprüchen 1 bis 5 oder eines Mittels gemäß Anspruch 6 oder 7 zur Bekämpfung von Hylotrupes bajulus und/oder Pyrrhidium sanguineum mittels der Paarungsstörungsmethode.

9. Verwendung von (3R)-3-Hydroxyhexan-2-on, einer Mischung gemäß Anspruch 4 oder 5 oder eines Mittels gemäß Anspruch 7 zur Bekämpfung von Hylotrupes bajulus und/oder Pyrrhidium sanguineum mittels der Monitor-, Abfang- oder Paarungsstörungsmethode.

10. Verfahren zur Herstellung einer Mischung zur Bekämpfung von Hylotrupes bajulus und/oder Pyrrhidium sanguineum gemäß den Ansprüchen 1 bis 5, dadurch gekennzeichnet, daß man ihre Komponenten getrennt herstellt und im geeigneten Verhältnis miteinander mischt.

11. Verfahren zur Bekämpfung von Hylotrupes bajulus und/oder Pyrrhidium sanguineum mittels der Paarungsstörungsmethode, dadurch gekennzeichnet, daß man eine Mischung gemäß den Ansprüchen 1 bis 5 oder ein Mittel gemäß Anspruch 6 oder 7 in einer solchen Menge anwendet, daß die weiblichen Tiere der Art bei der Auffindung der männlichen Tiere gestört werden.

12. Verfahren zur Bekämpfung von Hylotrupes bajulus und/oder Pyrrhidium sanguineum mittels der Monitormethode, dadurch gekennzeichnet, daß man (3R)-3-Hydroxyhexan-2-on, eine Mischung gemäß Anspruch 4 oder 5 oder ein Mittel gemäß Anspruch 6 oder 7 in einer solchen Menge anwendet, daß die weiblichen Tiere der Art angelockt werden.

13. Verfahren zur Bekämpfung von Hylotrupes bajulus und/oder Pyrrhidium sanguineum mittels der Abfangmethode, dadurch gekennzeichnet, daß man (3R)-3-Hydroxyhexan-2-on, eine Mischung gemäß Anspruch 4 oder 5 oder ein Mittel gemäß Anspruch 6 oder 7 in einer solchen Menge anwendet, daß die weiblichen Tiere der Art angelockt werden.

## Claims

1. A mixture comprising one stereoisomer of 3-hydroxyhexan-2-one and two stereoisomers of hexane-2,3-diol.

2. A mixture as claimed in claim 1, wherein the stereoisomer of 3-hydroxyhexan-2-one is (3R)-3-hydroxyhexan-2-one.

3. A mixture as claimed in claim 1 or 2, wherein the stereoisomers of hexane-2,3-diol are (2S,3R)-hexane-2,3-diol and (2R,3R)-hexane-2,3-diol.

4. A mixture of (2R,3R)-hexane-2,3-diol and (2R,3R)-hexane-2,3-diol.

5. A mixture as claimed in claims 1, 2 and 3, which comprises
a) from 70 to 100 parts by weight of (3R)-3-hydroxyhexan-2-one,
b) from 0.01 to 7 parts by weight of (2R,3R)-hexane-2,3-diol,
c) from 0.01 to 20 parts by weight of (2S,3R)-hexane-2,3-diol
and if desired
d) from 0.01 to 5 parts by weight of hexane-2,3-dione and/or
e) from 0.01 to 7 parts by weight of a racemic mixture of 2-hydroxyhexan-3-one.

6. A composition suitable for controlling Hylotrupes bajulus and/or Pyrrhidium sanguineum by the mating disruption method, containing an active amount of a mixture as claimed in claims 1 to 3 or a mixture of any two desired stereoisomers of hexane-2,3-diol and a liquid or solid carrier.

7. A composition suitable for controlling Hylotrupes bajulus and/or Pyrrhidium sanguineum by the monitoring, capture or mating disruption method, containing an active amount of (3R)-3-hydroxyhexan-2-one or a mixture as claimed in claim 4 or 5 and a liquid or solid carrier.

8. The use of a mixture as claimed in claims 1 to 5 or of a composition as claimed in claim 6 or 7 for controlling Hylotrupes bajulus and/or Pyrrhidium sanguineum by means of the mating disruption method.

9. The use of (3R)-3-hydroxyhexan-2-one, of a mixture as claimed in claim 4 or 5 or of a composition as claimed in claim 7 for controlling Hylotrupes bajulus and/or Pyrrhidium sanguineum by means of the monitoring, capture or mating disruption method.

10. A process for preparing a mixture for controlling Hylotrupes bajulus and/or Pyrrhidium sanguineum as claimed in claims 1 to 5, which comprises preparing its components separately and mixing them with one another in a suitable ratio.

11. A process for controlling Hylotrupes bajulus and/or Pyrrhidium sanguineum by means of the mating disruption method, which comprises applying a mixture as claimed in claims 1 to 5 or a composition as claimed in claim 6 or 7 in an amount such that the female animals of the species are disrupted in finding the male animals.

12. A process for controlling Hylotrupes bajulus and/or Pyrrhidium sanguineum by means of the monitoring method, which comprises applying (3R)-3-hydroxyhexan-2-one, a mixture as claimed in claim 4 or 5 or a composition as claimed in claim 6 or 7 in an amount such that the female animals of the species are attracted.

13. A process for controlling Hylotrupes bajulus and/or Pyrrhidium sanguineum by means of the capture method, which comprises applying (3R)-3-hydroxyhexan-2-one, a mixture as claimed in claim 4 or 5 or a composition as claimed in claim 6 or 7 in an amount such that the female animals of the species are attracted.

## Revendications

1. Mélange contenant un stéréo-isomère de la 3-hydroxy-hexan-2-one et deux stéréo-isomères de l'hexane-2,3-diol.

2. Mélange selon la revendication 1, caractérisé en ce qu'il s'agit, en ce qui concerne le stéréo-isomère de la 3-hydroxyhexan-2-one, de la (3R)-3-hydroxyhexan-2-one.

3. Mélange selon la revendication 1 ou 2, caractérisé en ce qu'il s'agit, en ce qui concerne les stéréo-isomères de l'hexane-2,3-diol, du (2S,3R)-hexane-2,3-diol et du (2R,3R)-hexane-2,3-diol.

4. Mélange du (2S,3R)-hexane-2,3-diol et du (2R,3R)-hexane-2,3-diol.

5. Mélange selon les revendications 1, 2 et 3, caractérisé en ce qu'il contient
a) à concurrence de 70 à 100 parties en poids, la (3R)-3-hydroxyhexan-2-one,
b) à concurrence de 0,01 à 7 parties en poids, le (2R,3R)-hexane-2,3-diol,
c) à concurrence de 0,01 à 20 parties en poids, le (2S,3R)-hexane-2,3-diol
et le cas échéant,
d) à concurrence de 0,01 à 5 parties en poids, l'hexane-2,3-dione et/ou
e) à concurrence de 0,01 à 7 parties en poids, un mélange racémique de la 2-hydroxyhexan-3-one.

6. Agent approprié pour lutter contre Hylotrupes bajulus et/ou Pyrrhidium sanguineum conformément au procédé de perturbation de l'accouplement, contenant une quantité efficace d'un mélange selon les revendications 1 à 3, ou encore un mélange de deux stéréo-isomères quelconques de l'hexane-2,3-diol et une substance de support liquide ou solide.

7. Agent approprié pour lutter contre Hylotrupes bajulus et/ou Pyrrhidium sanguineum conformément au procédé de surveillance, d'interception ou de perturbation de l'accouplement, contenant une quantité efficace de la (3R)-3-hydroxyhexan-2-one ou d'un mélange selon la revendication 4 ou 5 et une substance de support liquide ou solide.

8. Utilisation d'un mélange selon les revendications 1 à 5 ou d'un agent selon la revendication 6 ou 7 pour lutter contre Hylotrupes bajulus et/ou Pyrrhidium sanguineum au moyen du procédé de perturbation de l'accouplement.

9. Utilisation de la (3R)-3-hydroxyhexan-2-one, d'un mélange selon la revendication 4 ou 5 ou encore d'un agent selon la revendication 7 pour lutter contre Hylotrupes bajulus et/ou Pyrrhidium sanguineum au moyen du procédé de surveillance, d'interception ou de perturbation de l'accouplement.

10. Procédé pour la préparation d'un mélange pour lutter contre Hylotrupes bajulus et/ou Pyrrhidium sanguineum selon les revendications 1 à 5, caractérisé en ce qu'on prépare ses composants de manière séparée et on les mélange l'un avec l'autre dans le rapport approprié.

11. Procédé pour lutter contre Hylotrupes bajulus et/ou Pyrrhidium sanguineum au moyen du procédé de perturbation de l'accouplement, caractérisé en ce qu'on utilise un mélange selon les revendications 1 à 5 ou un agent selon la revendication 6 ou 7 en une quantité telle que les animaux femelles de l'espèce sont perturbés au cours de leur recherche des animaux mâles.

12. Procédé pour lutter contre Hylotrupes bajulus et/ou Pyrrhidium sanguineum au moyen du procédé de surveillance, caractérisé en ce qu'on utilise la (3R)-3-hydroxyhexan-2-one, un mélange selon la revendication 4 ou 5 ou encore un agent selon la revendication 6 ou 7 dans une quantité telle que les animaux femelles sont attirés.

13. Procédé pour lutter contre Hylotrupes bajulus et/ou Pyrrhidium sanguineum au moyen du procédé d'interception, caractérisé en ce qu'on utilise la (3R)-3-hydroxyhexan-2-one, un mélange selon la revendication 4 ou 5 ou encore un agent selon la revendication 6 ou 7 en une quantité telle que les animaux femelles sont attirés.
